(19) **Europäisches Patentamt**
**European Patent Office**
**Office européen des brevets**

(11) **EP 4 520 365 A1**

(12) **EUROPEAN PATENT APPLICATION**

(43) Date of publication:
**12.03.2025 Bulletin 2025/11**

(21) Application number: **23195311.8**

(22) Date of filing: **05.09.2023**

(51) International Patent Classification (IPC):
***A61M 5/20*** (2006.01)   ***A61M 5/24*** (2006.01)

(52) Cooperative Patent Classification (CPC):
**A61M 5/20; A61M 5/2422;** A61M 2205/3306;
A61M 2205/3334; A61M 2205/50

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Ypsomed AG**
**3401 Burgdorf (CH)**

(72) Inventors:
• **GLAUSER, Oliver**
  **3008 Bern (CH)**
• **MELLENBERGER, Andres**
  **3400 Burgdorf (CH)**

(74) Representative: **Meier Obertüfer, Jürg**
**Ypsomed AG**
**Brunnmattstrasse 6**
**3401 Burgdorf (CH)**

(54) **ADD-ON MODULE FOR AN INJECTION DEVICE**

(57) An add-on module (2) for an injection device comprising a dispense indicator (25) visible through a viewing window (10c) in a device housing (10) and the housing defines a longitudinal axis for the injection device. The add-on module (2) comprises an optical flow sensor (8) fixated to the add-on module and configured to track movement of the dispense indicator (25) relative to the housing (10) through the viewing window (10c). A processor programmed to run an algorithm enabling optical flow computation from optical flow sensor data and wherein the add-on module (2) is adapted to be i) releasably fixated to the housing (10) and ii) axially and rotationally fixated to the housing.

Fig. 12

D-D

EP 4 520 365 A1

**Description**

TECHNICAL FIELD

The current invention relates to an add-on module for an injection device.

BACKGROUND OF THE INVENTION

Injection or infusion devices such as injections pens, auto injectors, autopens, patch injectors or patch pumps use a drive mechanism for expelling medicament from the device. The drive mechanism comprises mechanical components for advancing, for example, a piston rod forwarding a bung in a reservoir or to drive a pumping mechanism. The injection device may include indicators such as for example, a status indicator, an indicator for displaying the amount of dosage set or delivered, an indicator for malfunctioning of the device, a progress indicator or a status indicator showing start/stop or progress of the injection or a dispense indicator indicating the status of the medicament dispense. The injection or infusion devices may be disposable or reusable after expelling the medicament.

There is a need to include additional features to those devices having mechanically driven indicators, for example by adding a module to the injection device. Preferably such an additional module is an electronic module and examples for those electronic modules may be a connectivity module with a transmitter and/or receiver to allow communication with other devices, a sensing module to allow for sensing patient parameters such as blood glucose values, a location module indicating geographical locations, a timer or calendar module, a communication or training module including a loudspeaker, or a sensing module to detect impact. Those additional electronic modules may be added as an add-on or supplementary device to the existing device. The analog information present on the indicator may be transformed into digital data by reading, scanning or detecting movement of the mechanically driven indicator.

The add-on device may be disposable or reusable. For a reusable add-on combined with a disposable injection or infusion device, the add-on must be removed from the disposable device for re-use of the add-on. For a disposable add-on it may be beneficial to separate the add-on from the mechanical components for separate waste treatment as the add-on devices include electronic components and batteries requiring a different treatment compared to the mechanical components or the components that are contaminated with medicament after use.

The add-on module may include a sensor or a sensor chip to read or scan the information available on the indicator, for example using OCR (optical character recognition) technology.

US 10,043,093 B2 discloses an injection pen with a rotatable dose dial sleeve including alphanumerical dose values printed in a helical manner onto the outside side surface. During dose setting, the dose dial sleeve is rotated and axially moved in the proximal direction thereby displaying the dose value in a viewing window in a side wall of a tubular housing. An add-on module is attachable at a specific angular position on the housing for covering the viewing window in the side wall. The add-on module includes a 2D sensor chip for recognizing images and an OCR processing algorithm is used to recognize the alphanumerical dose value set.

WO2013120775 discloses a clamping mechanism for attaching an add-on module to the housing of an injection pen, the add-on module is configured to scan the dose values printed on a rotatable dose dial sleeve.

US 2022/0016352 A1 discloses an attachment mechanism for attaching an add-on module to the proximal end of a rotatable dose dial sleeve such that the add-on co-rotates together with the dose dial sleeve. The attachment mechanism includes a sleeve and a clamp. Moving the sleeve over the clamp deforms the clamp such that the assembly is fixated to the dose dial knob.

There is a need for a versatile and simple add-on module overcoming the drawbacks of the past modules that may be attached at any position at the proximal end of the housing of the injection device and with a sensor that can track movement of the indicator. The sensor may even track movement of the indicator even if no values are printed onto the indicator.

This objective is solved by the independent claim and specific variants are disclosed in the dependent claims. Furthermore a system including the add-on module and an injection device is claimed.

DEFINITIONS

The term "medicament" or "medication" includes any flowable medical formulation suitable for controlled administration through a means such as, for example, a cannula or a hollow needle and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from -or harvested by- biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances.

The distal end or distal direction is defined by the direction of the hollow needle configured to penetrate the skin of the patient. For an injection device such as an injection pen this may be the injection needle and the end of the pen holding the needle or being configured to hold the needle is the distal end. For an infusion device the distal end and the distal direction is towards the needle configured to penetrate the skin of the patient, which may be along the axis of the device or tilted or perpendicular to the axis of the device. The distal direction in an infusion device represents the direction in which the medicament flows towards the insertion needle. The proximal direction or end is opposite to the distal direction or end.

The PCB in the context of this invention may relate to the board itself or to the board including all electronic components. Optionally, the battery is part of the PCB as well.

In the claims, the word "comprising" does not exclude other elements or steps, and the indefinite article "a" or "an" does not exclude a plurality. For example "an attachment member comprises an elastic arm" does not exclude the fact that there may be two elastic arms or a plurality of elastic arms that are part of the attachment member and functionally or structurally fulfill the purpose of "an elastic arm". The mere fact that certain elements or steps are recited in distinct claims shall not preclude the existence of further meaningful combinations of these elements or steps.

Optical flow or optic flow is the pattern of apparent motion of objects, surfaces, and edges in a visual scene caused by the relative motion between an observer and a scene. Optical flow sensors are extensively used in computer optical mice for tracking motion of a mouse across a surface,

GENERAL DESCRIPTION OF THE INVENTION

In the current invention an add-on module for an injection device is disclosed including a dispense indicator visible through a viewing window in a device housing. The housing defines a longitudinal axis for the injection device and the add-on module includes an optical flow sensor fixated to the add-on module and configured to track movement of the dispense indicator relative to the housing through the viewing window. The add-on module further includes a processor programmed to run an algorithm enabling optical flow computation from optical flow sensor data received from the sensor. The add-on module is adapted to be i) releasably fixated to the housing, and ii) axially and rotationally fixated to the housing.

The dispense indicator, may also be a progress indicator, a status indicator, a dose dialed indicator, a dose dispensed indicator or an error indicator. Dispense indicator may be adapted to be rotated relative to the housing before, during or after an injection. Alternatively the dispense indicator moves axially without rotation or moves by a combined axial/rotational movement. The movement of the dispense indicator may temporarily pause during the injection.

The dispense indicator moves relative to the viewing window before, during or after injection and can be viewed by the user when no add-on module is attached or can be viewed again when the add-on is removed from the injection device.

The add-on module may be a supplementary device for the injection device and may be releasable attacheable to the same injection device or may be used on another injection device once the first injection device has been emptied. The another device is preferably the same device as the first device. The add-on may be used on a plurality of injection devices and the processor may include a counting unit for counting the number of injection devices for therapy management.

The optical flow sensor is configured to track movement of the dispensing indicator when the add-on module is attached to the injection device. The optical flow sensor may be a tracking sensor tracking displacement of subsequent images or frames taken by a 2D sensor chip. The optical flow sensor is preferably not an Optical Character Recognition (OCR) sensor. The sensor may not be capable to detect black and white pixel determination.

The optical flow sensor may be capable to detect start and stop for the movement of the dispense indicator, or track the distance travelled by the dispense indicator.

The dispense indicator may continuously rotate during an injection and the same position on the indicator may repeatedly be viewed by the user, and tracked in the viewing window by the flow sensor of the add-on device. The optical flow sensor may calculate the total distance travelled by a position on the surface of the dispense indicator even when this position on the indicator is repeatedly viewed or scanned in the viewing window. The optical flow sensor data may be based on a sequence of subsequent frames, pictures or images taken by the 2D sensor chip.

Optical flow computation may use the sensor data such as positions or structures extracted from subsequent frames and the respective shift of positions or pixel shifts are input data for the algorithm. The processor is programmed to run the algorithm using the sensor data. The processor may be programmed to calculate the dose selected or dispensed from the distance travelled by the dispense indicator and may be programmed to present an alarm if the distance travelled is different from a predetermined value stored in a memory module.

The add-on module includes a power source such as a battery and may include a communication module with a transceiver, for example a Bluetooth module, for communication with an external device. The external device may be a smart phone or a tablet computer, a wearable or a cloud server.

The add-on module may include an optical display for displaying information to the user as the dispense indicator is at least partially covered by the add-on module. The optical display may be viewed from the proximal end of the add-on module. The information may be based on the optical flow computation for the movement of the dispense indicator. The display may be a Liquid Crystal Display (LCD) or an Organic Light Emitting Diode (OLED) display. The display may include a touch sensitive screen. The add-on module may include a signaling element such as a Light Emitting Diode (LED) for signaling the start, the stop or progress of the injection. The add-on module may include a load speaker or buzzer for acoustic signaling the start, stop or progress of the injection. The signaling element may be used to signal an error for the injection.

The optical flow sensor of the add-on module may be adapted to track helical movement of the dispense indicator. The helical movement is relative to the housing and around the longitudinal axis. Alternatively, the optical flow sensor may be adapted to track an axial movement of the dispense indicator along the longitudinal axis without rotational movement.

The optical flow sensor may be adapted to track a continuous rotational movement of the dispense indicator around the longitudinal axis without axial movement relative to the housing. The injection devices that may be used in combination with the add-on module include a dispense indicator that performs a helical movement relative to the housing or an axial movement relative to the housing or a rotational movement without an axial movement.

The add-on module may be adapted to be axially fixated to a proximal end of the housing of the injection device and rotationally fixated at any angular position around the longitudinal axis of the housing. The axial position of the add-on module relative to the dispense indicator may be fixed whereas the rotational position of the add-on module (and thus the sensor) may be variable. The add-on module may be re-fixated to the same injection device on a different angular position and the add-on remains capable detecting or tracking the movement of the dispense indicator. The add-on module may be re-fixated on another injection device at a different angular position compared to the first device and still be able to track movement of the dispense indicator. Optionally, the add-on module may be adapted to be fixated at a plurality of defined axial positions around the longitudinal axis of the housing.

The processor in the add-on module may be adapted to compute the number of rotations of the dispense indicator. The processor in the add-on module may be adapted to transfer the number of rotations into information that may be displayed or sent or communicated to the user. The processor may use calibration data stored in a memory for transferring the rotations in data that may be transmitted to an external device or displayed on the display. The data transmitted or displayed may relate, for example, to the type of device used, the expiry date for the device, the start/stop or the progress of the injection, the dose set, or the dose dispensed. The data transmitted may include an error message if, for example the time used for dispensing the medicament exceeds a stored value.

The add-on module may include a timer module capable to detect the start, stop and duration of the injection. The timer may be started by the add-on module when the sensor starts collecting data from the dispense indicator and may stop the timer when the dispense indicator stops moving. The injection time may be displayed on the display and/or transmitted to an external device. The timer module may indicate, display or acoustically announce to the user when the injection device may be removed from the patient after completion of the injection using a post injection delay time to ensure that all medicament can be absorbed at the injection site.

The dispense indicator for the injection device may include non-alphanumeric patterns printed onto the surface or the dispense indicator does not include any visible pattern on the surface. The optical flow sensor may be adapted to track movement of a dispense indicator including non-alphanumeric patterns on the surface or to track movement of a dispense indicator without a visible pattern on the surface. The surface may be a polished and shining surface. The pattern may be a hatching with a fixed spacing or a variable line spacing. The pattern may be printed black on a white surface. The pitch of the hatching may be constant or variable. The pattern may be provided as a textured surface, for example as a roughened hatching on a polished background. The pattern may further include a two dimensional (2D) or three dimensional (3D) bar code or data matrix code or QR code. The 2D or 3D codes may provide further information for the optical flow sensor or for another sensor in the add-on capable of reading 2D or 3D matrix codes. The 2D or 3D matrix codes may include coded data relate to the manufacturing date of the device, the lot number, the medicament contained in the injection device or the expiry date for the mechanical parts of the injection device or the expiry date for the medicament contained in the injection device. The 2D or 3D matrix codes may be viewed by the user at the start or at the end of the injection and are therefore available for the optical flow sensor or for another sensor at the start or at the end of the injection.

The dispense indicator may include both a non-alphanumeric pattern and alphanumeric numbers or alphanumeric characters. The alphanumeric characters may, for example, include a company name. The pattern on the dispense indicator may include an icon or a logo related to the manufacturer of the injection device or to a brand name for the medicament.

Moreover, the dispense indicator may include a binary code and in particular a reflected binary code such as, for example, a Gray code allowing an absolute position determination. A reflected binary code prevents errors or ambiguities during transition from one number to the next since only one bit changes its value during a transition between two adjacent numbers.

The viewing window for the injection device may be provided as a transparent circumferential rim at the proximal end face of the injection device covering a disk shaped dispense indicator rotatably mounted behind the transparent circumferential rim. Alternatively, the viewing window is located in a side wall of a pen shaped injection device and the sensor is capable detecting images from a lateral perspective.

The viewing window may be an opening in the housing or in a housing part. The viewing window may be covered by a transparent cover, for example by a cover made from a transparent plastic such as polymethylmethacrylate (PMMA) or polycarbonate (PC) or polystyrene (PS).

The optical flow sensor for the add-on module may include a 2-Dimensional image sensor chip and an infrared laser light source and the optical flow computation uses the displacement between subsequent images of the dispense indicator taken by the image sensor. The wavelength of the laser light used may vary between 700nm and 1050nm. A LED source may be used emitting laser light with a wavelength of 850nm. The 2-Dimensional sensor chip may capture a sequence of ordered images allowing the estimation of motion of the dispense indicator.

The add-on module may further include a fixation sleeve adapted to be arranged over the proximal end of the housing of the injection device and an attachment member that is coupled to, and arranged within the fixation sleeve. The attachment member may be co-axially arranged within the fixation sleeve. The attachment member is adapted to grip the proximal end of the housing, or the viewing window. The fixation sleeve is adapted to move relative to the attachment member from a first position allowing the dispense indicator to be moved with respect to the attachment member to a second position inhibiting relative movement between the dispense indicator and the attachment member. The movement may be inhibited as a threshold force for moving the fixtion sleeve relative to the attachment member may be overcome first. The add-on module is fixated to the housing via the attachment member when the fixation sleeve is in the second position. The fixation is a releasable fixation in the that the add-on module may be removed if the fixation sleeve is moved back to the first position. The attachment member is adapted to be deformed during movement of the fixation sleeve from the first to the second position thereby fixating the add-on module to the proximal end of the housing. The relative movement between the fixation sleeve and the attachment member may be an axial movement or a rotational movement or a screw type of movement.

The optical flow sensor is axially and rotationally fixated to the add-on module. The optical flow sensor may be fixated to the fixation sleeve and/or to the attachment member.

In the first position of the fixation sleeve, the proximal end of the injection device or the dispense indicator with the viewing window may be inserted into the attachment member of the add-on module. The flexible element of the attachment member may deform during insertion and click into receiving members on the housing for positioning the add-on module on the housing without fixating the add-on to the housing as the fixation sleeve is in the first position. The flexible element may deform again for detachment when the fixation sleeve is in the first position.

In the first position of the fixation sleeve, the attachment member may be at least partially visible, or not fully covered by the fixation sleeve and in the second position the attachment member may not be visible and fully covered by the fixation sleeve.

The attachment member is coupled to the fixation sleeve by an axial, rotational, bayonet or a screw type of coupling. The axial coupling is adapted to allow for an axial movement of the fixation sleeve relative to the attachment member. The axial movement may be guided by guide slots engaging guide keys on both parts and axial stops limit the relative movement between the attachment member and the fixation sleeve between the first and second positions. A bayonet type of coupling provides an axial movement between the first and second position for the fixation sleeve followed by a rotational movement relative to the attachment member for fixating the add-on to the housing of the injection device.

The attachment member may include several sections, a section for holding the sensor, a section for engaging the fixation sleeve and a gripping section including gripping elements for gripping the housing of the injection device. The attachment member is preferably manufactured as a single part from a stamped metal sheet or from an injection moulded plastic part.

The attachment member or the gripping section of the attachment member includes at least one elastic arm adapted to flex towards the housing of the injection device as the fixation sleeve moves from the first position to the second position, preferably moves axially from the first to the second position. The flexing towards the housing may also be induced by a relative rotational movement. The attachment member may include a plurality of elastic arms for engaging the housing and the attachment member may include a circumferential rim and the elastic arms may depend from the rim. The elastic arms may depend in the distal direction for engaging the housing of the injection device thereby providing the gripping section. Connecting elements may depend from the circumferential rim and point into the proximal direction for engaging the fixation sleeve. The connecting elements may include the guide element or the key for guiding the relative movement between the fixation sleeve and the attachment member.

A protrusion or hook, or an edge or a projection at the distal end of the elastic arm in the gripping section may be adapted to engage a recess, an indention or opening or a circumferential recess at the proximal end of the housing. The recess or circumferential recess may be located at the proximal end of the injection device for example between a main housing and the viewing window or the transparent cover covering the dispense indicator. The circumferential recess may be continuous or may include a plurality of recessed sections all located at the same axial position along the longitudinal axis and each section is adapted to engage at least one protrusion or hook. The engagement between the protrusions and recess may be a friction fit engagement. There may be a plurality of continuous recesses located adjacent to another and each circumferential recess is positioned at a fixed axial position along the longitudinal axis. The plurality of adjacent recesses may engage a plurality of hooks or protrusions located adjacent to another on each flexible arm, in this case there may be a form fit engagement between the plurality of recesses and the plurality of protrusions or hooks. The surface within the recess may be a textured surface, a rippled surface or a roughened surface increasing friction between the protrusion and the circumferential recess when the add-on is fixated to the housing. Increased friction may improve the fixation between the add-on and the housing and prevent relative rotation between the add-on and the injection device when fixated. The tip of the protrusion at the end of the flexible arm may be textured or roughened as well complementary to the textured surface of the recess.

The fixation sleeve includes a fixation member protruding from an inner surface of the fixation sleeve and may include a ramped surface for engaging the elastic arm. The ramped surface of the fixation member may engage a complementary ramped surface on the elastic arm providing a gearing engagement during movement of the fixation sleeve from the first to the second position. The ramped surface and the complementary ramped surface may slide along each other such that the elastic arm is deflected radially inwards. The ramped surfaces may provide that the protrusions on the elastic arms on the attachment member snap-fit or click into the recess during or after moving the fixation sleeve into the second position.

A further aspect of the invention relates to a system including the add-on module and an injection device comprising the dispense indicator. The system may be provided as a kit of parts. The add-on module may be available as a pre-attached supplementary device onto the injection device or the add-on module is provided as a separate unit that the user attaches to the injection device.

The injection device may be a pen-shaped injection device and the longitudinal axis of the housing equals or is parallel to the pen axis.

The injection device may be an auto injector or an autopen. The auto injector may include an injection spring for advancing a plunger rod for expelling medicament from the injection device during an injection and the injection spring may be configured to continuously rotate or intermittently rotate the dispense indicator during the injection. The viewing window for the dose dispense indicator may be located at a proximal face of the auto injector or at a lateral wall of the auto injector. The viewing window may be covered by a transparent cover or the viewing window may be a passage in the housing.

The injection device may be a variable or fixed dose injection pen in another embodiment of the invention. The injection pen may include a dose dial sleeve that is manually rotated for setting of a dose and the outer surface of the dose dial sleeve provides the dispense indicator, for example by dose values, icons or patterns printed onto the surface of the dose dial sleeve. The viewing window for the dose dispense indicator may be located at a lateral wall of the pen shaped injection device.

The dispense indicator for the injection devices may include non-alphanumeric patterns printed onto the surface or the dispense indicator does not include any visible pattern on the surface.

LEGENDS TO THE FIGURES

While the invention has been described in detail in the drawings below and foregoing general description, such description is to be considered illustrative or exemplary and not restrictive. Variations to the disclosed embodiments can be understood and effected by those skilled in the art and practicing the claimed invention, from a study of the drawings, the disclosure, and the appended claims.

Figure 1:     Exploded view for an autoinjector,
Figure 2:     Longitudinal sections for the autoinjector. Two sections shown taken in planes oriented perpendicular to another,
Figure 3:     Top and bottom view for the dispense indicator with a hatching pattern on the outside surface,
Figure 4:     Top view for the dispense indicator according to another embodiment with a different hatching pattern compared to Figure 3,
Figure 5:     Top view for the dose indicator having no pattern on the outside surface,
Figure 6:     Proximal section for the auto injector with a circumferential viewing window and a circumferential recess,
Figure 7:     Proximal section for the auto injector, add-on module positioned over the distal end and fixation sleeve in the first position,
Figure 8:     Proximal section for the auto injector, add-on module fixated to the distal end and fixation sleeve in the second position,
Figure 9:     Fixation sleeve for the add-on module,
Figure 10:    Attachment member for the add-on module,
Figure 11:    Longitudinal section for the assembly of the fixation sleeve and the attachment member,

Figure 12: Cross section for the assembly of the fixation sleeve and the attachment member,
Figure 13: Longitudinal section for the assembly of Figure 11 in a different plane with the fixation sleeve in the first position,
Figure 14: Longitudinal section for the assembly of Figure 11 in a different plane with the fixation sleeve in the second position,
Figure 15: Longitudinal section for the add-on module positioned over the proximal end of the auto injector; fixation sleeve in the first position,
Figure 16: Longitudinal section for the add-on module positioned over and fixated to the proximal end of the auto injector; fixation sleeve in the second position,
Figure 17: Proximal section for the auto injector, add-on module according to a second embodiment, fixation sleeve in the first position,
Figure 18: Proximal section for the auto injector, add-on module according to a second embodiment, fixation sleeve in the second position,
Figure 19: Schematic representation for the position of the optical flow sensor with respect to the viewing window of the auto injector,
Figure 20: Graphical representation for the detection of pixel shifts for the rotating dispense indicator versus time,
Figure 21: Graphical representation showing the transformation of the pixel shifts into number of rotations using optical flow computation.

DETAILED DESCRIPTION OF THE FIGURES

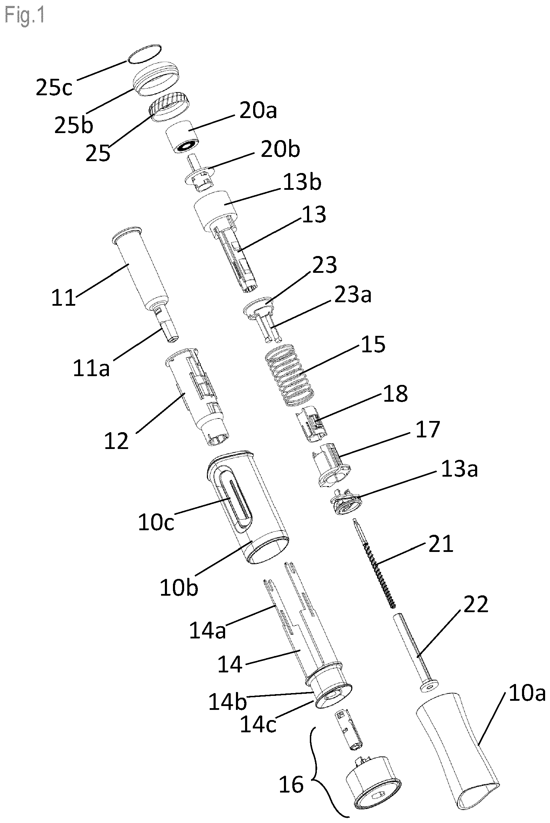

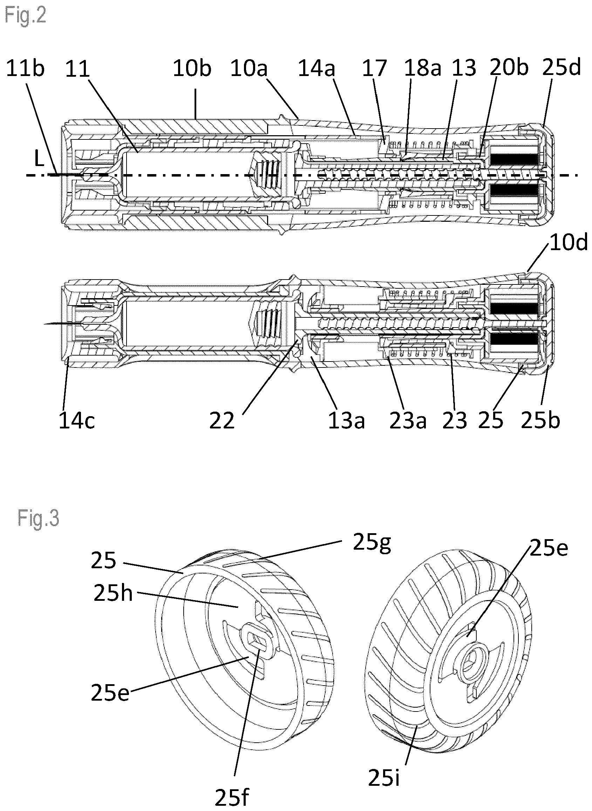

Figure 1 presents an explosive view of an auto injector that may be used in combination with the add-on module. Figure 2 shows two longitudinal section of the auto injector that are oriented perpendicular to another. The auto injector presented in Figure 2 presents a status after cap removal and needle insertion in the patients' skin. The injection has not been started yet.

The auto injector includes a sleeve shaped housing 10 defining a longitudinal axis L (Figure 2). The sleeve shaped housing 10 includes a distal housing part 10b and a proximal housing part 10a. The proximal housing part 10a cannot be separated from the distal housing part 10b after assembly. The proximal housing part 10a may include a structured outer surface, for example a rippled surface, for securely holding the auto injector. A medicament reservoir is provided, for example as a prefilled syringe 11 including stacked hollow needle at the distal end and the prefilled syringe can be inserted into a syringe holder 12. The syringe holder 12 is configured to be rotationally and axially secured to the housing 10. The prefilled syringe 11 is biased in the distal direction by a holding/spring element 13a thereby pushing the body of the prefilled syringe, or a shoulder section of the prefilled syringe, in abutment with a shoulder section of the syringe holder. The prefilled syringe 11 is axially arranged with respect to the distal housing 10b such the tip of the needle may extend from the distal end of the distal housing part 10b or from the distal end of a needle cover sleeve 14 when the needle cover sleeve 14 has been moved to a retracted position. The protruding distance for the needle defines the subcutaneous or intramuscular needle penetration depth for the auto injector. The needle cover sleeve 14 covers and at least laterally protects the tip of the needle before and after the injection. The needle cover sleeve 14 may be moved along the longitudinal axis L against the bias provided by a needle cover spring 15 over an actuation distance from an extended position to the retracted position thereby starting the injection. The needle cover sleeve 14 includes two arms 14a which are rotated over an angle of 90 degrees with respect to the longitudinal axis of the housing 10 such that a viewing window 10c is not blocked by the presence of the two arms 14a. The two arms 14a of needle cover sleeve 14 are attached to a sleeve shaped distal section 14b. The sleeve shaped distal section 14b may have an elliptical cross section and a flange 14c is located at the distal end having an elliptical shape configured for engaging the skin of a patient. After the injection the needle cover sleeve 14 may be moved distally by the bias provided by the needle cover spring 15 from the retracted position back to the extended position or needle cover position. The auto injector may include a lock out mechanism which locks the needle cover sleeve 14 with respect to the housing after the injection has been completed such that the needle cover may not be moved again to the retracted position.

A power package includes a spring, for example a spiral spring 20a and a spring reel 20b. One end, or outer end of the spiral spring 20a is connected to a spring sleeve 13b. The spring sleeve 13b is part of a mechanic holder 13 which is secured to the housing or may be integrated into the housing. The other end, or the inner end of the spiral spring 20a is rotationally secured to the spring reel 20b. The spring reel 20b includes a spring shaft and a distal spring flange. The spiral spring 20a or rather the spring reel 20b may rotate a drive element or threaded rod 21 for axially advancing a plunger rod 22. The plunger rod may be advanced with rotation or without rotation. For the latter option, thread segments on the outer surface of the threaded rod 21 engage thread segment on the inside of the hollow plunger rod 22. The plunger rod 22 is rotationally secured with respect to the housing while allowing axial movement. The threaded engagement between the threaded rod 21 and the plunger rod 22 extends axially over a distance such that the plunger rod may be moved along the longitudinal axis for a dose delivery stroke to expel medicament from the prefilled syringe.

The prefilled syringe 11 includes a cylindrical barrel for holding the medicament and a hollow needle is attached to the distal end of the barrel via a shoulder section. The hollow needle allows for expelling medicament from the syringe. The injection needle of the prefilled syringe is covered by a needle shield 11a which may be a soft or Rigid Needle Shield (RNS). The rigid needle shield includes an elastic core element covering and securing the tip of the needle enclosed by a shell made from a non-elastic plastic material. The needle shield 11a protects the medicament contained within the barrel of the prefilled syringe from contamination and retains the hollow needle in a sterile condition. A cap remover 16 is located at the distal end of the auto injector when the auto injector is in the initial state. The cap remover 16 includes two components, a distal component that can be gripped by the user and a proximal sleeve shaped component engaging the needle shield 11a (Figure 1). The user can grip the cap remover 16 and axially and/or rotationally remove the cap remover from the auto injector. The cap remover 16 has been removed in Figure 2 thereby also removing the needle shield 11a such that the needle of the prefilled syringe is available for needle insertion.

A switching sleeve 17 is coupled to the proximal end of the arms 14a of the needle cover sleeve 14 in a form fit engagement. The switching sleeve 17 is preferably snap-fitted onto the proximal end of the arms 14a. A distal end of the needle cover spring 15 at least partially surrounds the switching sleeve 17 and a distal end of the needle cover spring 15 abuts a flange on the switching sleeve 17 (Figure 2). The movement of the switching sleeve 17 in the distal direction is limited by the holding element 13a. The holding element 13a is snap fitted to the distal end of the mechanic holder 13 after assembling the switching sleeve 17 to the arms 14a of the needle cover sleeve 14. A locking sleeve 18 is coaxially arranged within the switching sleeve 17. The locking sleeve 18 includes a locking member 18a located at a distal end of a flexible arm of the locking sleeve 18 and the locking member 18a engages the switching sleeve 17 such that a proximal movement of the needle cover sleeve 14 is transferred to a proximal movement of the switching sleeve 17 (via the above mentioned form fit engagement) and from the switching sleeve 17 to the locking sleeve 18 via the locking member 18a (Figure 2). An additional proximal latching distance for the locking sleeve 18 relative to the switching sleeve 17 is provided whereby the locking sleeve 18 is moved in a proximal end position ensuring that the locking member 18a is securely released from the switching sleeve and the flexible arms including the locking member 18a may be moved inwards. The bias provided by the flexible arms on the locking member 18a ensures that the locking member 18 engages a proximally directed ridge or a recess and latches the locking sleeve for a distal movement with respect to the housing. The switching sleeve 17 is moved distally over the (latched) locking member 18a by the needle cover spring 15 when the auto injector is removed from the skin. The resilience of the flexible arms ensures that the locking member 18a is moved in a locking position and the locking member 18a engages a proximally directed ridge on the switching sleeve 17 such that the switching sleeve 17 and therewith also the needle cover sleeve 14 are locked and prevented from being again moved in the proximal direction.

A coupling sleeve 23 with two holding elements 23a is rotationally coupled to the spring reel 20b via coupling elements. The two holding elements 23a engage recesses in the mechanic holder 13 and are prevented from a radial outward movement by an inner surface of the locking member 18. The mechanic holder 13 is fixated to the housing and axial movement of the coupling sleeve 23 is prevented by the holding elements 23a engaging the locking sleeve 18 and the mechanic holder 13. The locking sleeve 18 is moved proximally by the needle cover sleeve 14 when triggering the injection such that the holding elements 23a are free to move radially outward from the recesses in the mechanic holder 13 thereby releasing the coupling sleeve 23. The coupling sleeve can move in the proximal direction thereby decoupling the coupling elements between the coupling sleeve and the spring reel 20b. The spring reel 20b is biased by the spiral spring 20a and starts rotating. The spring reel 20b includes an axial passage for the threaded rod 21 and the shape of the passage accommodates the outer dimensions of the threaded rod 21 such that the threaded rod 21 is rotationally coupled and axially slidable with respect to the spring reel 20b. Rotation of the spring reel 20b by the spiral spring 20a also rotates the threaded rod 21. The external thread segments on the outer surface of the threaded rod 21 engage the threading on the inside of the hollow plunger rod 22 and the plunger rod is axially advanced without rotation as the plunger rod 22 is rotationally secured to the housing. The plunger rod 22 abuts a piston in the prefilled syringe and advancement of the piston expels medicament through the injection needle.

The auto injector includes an indicator 25, for example a dispense indicator located at the proximal face of the auto injector behind a transparent cover 25b. A non-transparent disk 25c covers the central part of the transparent cover 25b thereby leaving a transparent circumferential rim shaped viewing window 25d. The user can view the dispense indicator 25 through the viewing window 25d as the dispense indicator moves or rotates during an injection.

The transparent cover 25b may be attached, for example snap fitted, glued or welded to the proximal end of the proximal housing 10a. A circumferential recess 10d between the proximal housing 10a and transparent cover 25b is available for attachment of an add-on device as will be explained below.

Two perspective views for the dispense indicator 25 from the top and bottom are depicted in Figure 3. The dispense indicator 25 includes a skirt 25g that extends distally from a bottom surface 25h. The bottom surface 25h includes a plurality of flexible arms 25e that extend in the proximal direction when the dispense indicator 25 has been assembled into the auto injector. The bottom surface furthermore includes a passage or recess 25f. A pattern 25i may be printed, embossed, engraved, laser marked or overmolded on the outside surface of the dispense indicator 25. The pattern may be on the outside surface of the skirt 25g and may extend towards the bottom surface of the dispense indicator 25. The pattern may include different color codings for indicating the start position, progress and final position of the dispense indicator and therewith provide a visual status indicator for the auto injector. The indicator may be capable to indicate a malfunctioning of the auto injector, for example a premature stop of the injection when the prefilled syringe has not been emptied yet. The pattern 25i may include a hatching and the hatching may have a constant spacing or a variable spacing. The lines of the hatching may all have the same color in one embodiment. The lines may have alternating colors in another embodiment. The colors used in the embodiments may provide photoluminescence to the indicator 25 providing an indicator that may be viewed in the dark. The pitch of the hatching may be constant in another embodiment or the pitch may vary around the circumference in an alternative embodiment. The hatching may be combined with other patterns. In an alternative embodiment, the indicator 25 includes alphanumeric values printed onto the surface. The pattern may thus include a combination of a hatching, numbers and letters. The alphanumeric numbers used may be used to display a brand name for the product to be injected or a company name producing the medicament and/or the auto injector or for displaying instructions how to use the auto injector or how to discard the auto injector after use.

The pattern 25i may be a 1-dimensional or 2-dimensional code language such as, for example a bar code or a QR code. Alternatively, the indicator 25 may not have a pattern printed onto the outside surface.

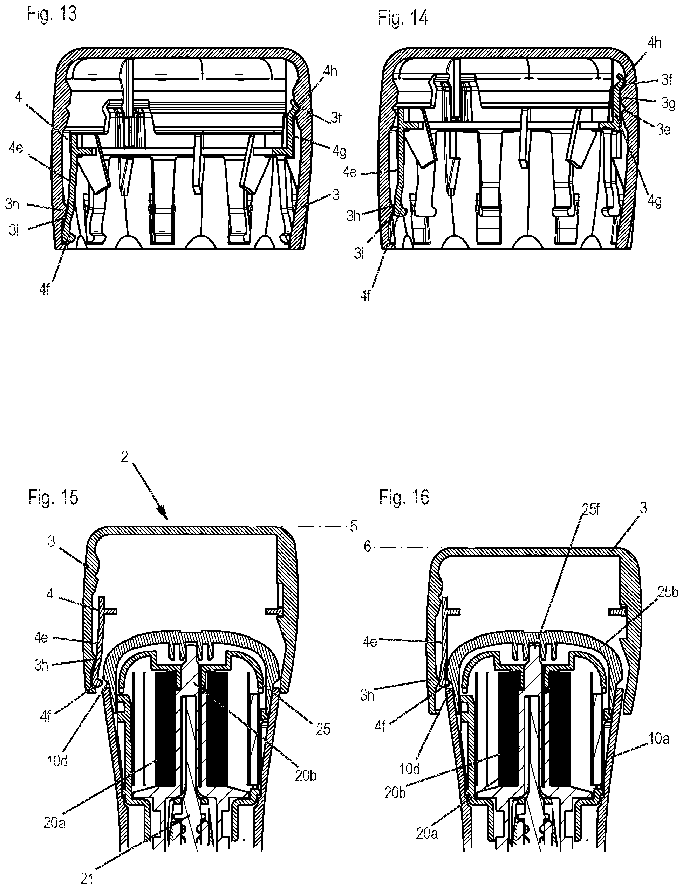

The passage of recess 25f is configured to engage the proximal end of the threaded rod 21. The passage may have a rectangular shape or shaped with a cross. The outer shape of the proximal end of the threaded rod 21 may fit into the passage 25f such that a rotation of the threaded rod 21, for example during the injection, is transferred into a rotation of the indicator 25. The user thus can view the indicator rotating during the injection. The indicator starts rotating at the start of the injection and stops rotating at the end of the injection. The indicator 25 may be keyed to another element of the auto injector that rotates during the injection such as for example, the spring reel 20b. The spring reel 20b may include a proximally directed protrusion engaging the passage or recess 25f in the indicator 25. In Figure 2, the threaded rod passes through a passage in the spring reel 20b, alternatively, the spring reel 20b directly engages the indicator 25 as will be presented below in Figure 15.

The indicator 25 is driven by the spring force provided by the spiral spring which drives the spring reel 20b and the threaded rod 21. The rotation of the indicator 25 may be geared up or geared down by a gearing mechanism present between the dispense indicator 25 and the spring reel 20b or between the dispense indicator and the threaded rod 21. The gear mechanism may include a plurality of gear wheels operatively positioned between the indicator 25 and the spring reel 20b or the threaded rod 21.

The dispense indicator may continuously rotate during the injection and the dispense indicator may rotate once, twice or a plurality of times around the longitudinal axis of the auto injector. The flexible arms 25e in the bottom surface 25h extend in the proximal direction and may ratchet against distally oriented protrusions on the inner surface of the transparent cover 25b for producing an audible and/or tactile feedback during the injection.

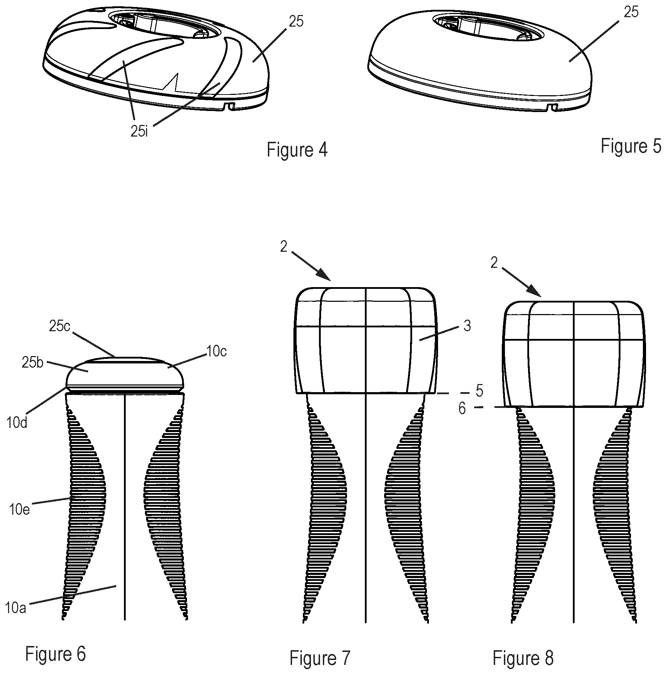

Further embodiments for the dispense indicator 25 are presented in Figures 4 and 5 having a hatching 25i with a different spacing compared to the embodiment presented in Figure 3. As an alternative, the surface of the dispense indicator 25 may have no pattern and have a polished surface (see Figure 5).

The proximal section for the auto injector without an add-on device is shown in Figure 6. The proximal housing 10a includes a structured or rippled gripping surface 10e. The circumferential recess 10d located between the proximal end of the housing 10a and the transparent cover 25b is available for attaching an add-on module 2. The non-transparent disk 25c covers the proximal face of the transparent cover 25b providing the viewing window 10c as a transparent circumferential rim. The add-on module is first positioned over the proximal end of the auto injector as shown in Figure 7. The add-on module includes a fixation sleeve 3 that can be moved axially with respect to the housing of the auto injector from a first position 5 to a second position 6 (Figure 8). In the first position 5 for the fixation sleeve 3, the add-on module 2 can be positioned over the proximal end of the auto injector (Figure 7) whereas the add-on module 2 is fixated to the proximal end of the auto injector when the fixation sleeve has been moved to the second position 6. The movement of the fixation sleeve from the first to the second position is a pure axial movement along the longitudinal axis L.

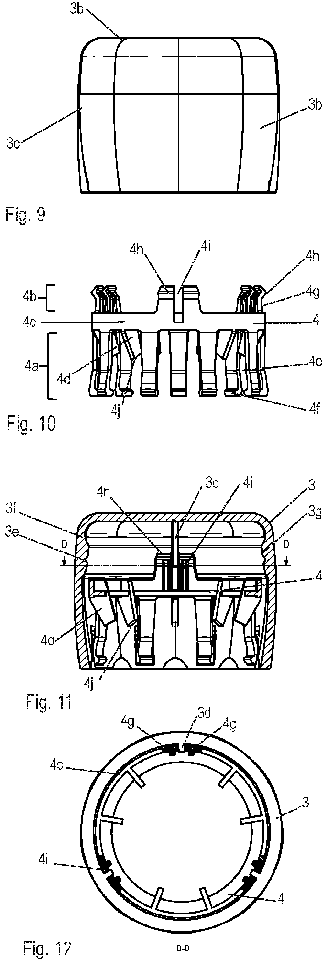

The components for the add-on module 2 are presented in Figures 9 and 10. The fixation sleeve 3 includes a proximal end wall 3b and a cylindrical side wall 3c. The side wall 3c and proximal end wall 3b form a bore for receiving an attachment member or clamp 4. The attachment member or clamp 4 that can be inserted into the bore is presented in Figure 10. The proximal end wall may be constructed from a transparent material allowing for displaying information provided by LED lights or by a display module located behind the end wall. The attachment member 4 includes a gripping section 4a adapted to engage and fixate to the proximal end of the housing of the injection device, such as to the circumferential recess 10d. A holding section 4b is connected to the gripping section 4a via a circumferential rim 4c. The holding section 4b provides for the engagement to the fixation sleeve 3. A plurality of protrusions 4d protrude from the circumferential rim 4c in to the distal direction. The plurality of protrusions 4d include a distal end surface 4j configured to abut the outer surface of the transparent cover 25b and/or the disk 25c of the auto injector. The space between a pair of the protrusions 4d may be available as a platform for holding an optical flow sensor. The inner space of the holding section 4b and/or the circumferential rim 4c may be available for holding or attaching other modules of the add-on module such as, for example, a power supply module including a battery, a processing module including an integrated circuit, a memory or storage module, a communication module, or a display module. The circumferential rim 4c may act as a mechanics holder for holding a printed circuit board (PCB) that may provide a platform for the above mentioned modules.

The gripping section 4a includes a plurality of flexible arms 4e attached to, and protruding in the distal direction from the circumferential rim 4c. A hook or protrusion 4f is located at the distal end of each flexible arm 4e and the protrusions 4f are oriented towards the center of the attachment member 4. The holding section 4b includes a plurality of proximal arms 4g attached to the circumferential rim 4. The arms 4g are oriented in the proximal direction and each arm includes a protrusion 4h oriented outwards from the center of the attachment member 4. The plurality of arms 4g are paired and an axial groove 4i is provided between each pair of the proximal protrusions 4h.

A longitudinal section for the assembly of the attachment member 4 and the fixation sleeve 3 is presented in Figure 11. The attachment member or clamp 4 being fully inserted into the bore provided by the fixation sleeve 3. The outer wall 3c covers the attachment member when the fixation sleeve is in the first position and in the second position.

Engagement between the fixation sleeve and the attachment member:

The fixation sleeve 3 includes an axial key 3d engaging the groove 4i, see Figure 11. The key-groove engagement rotationally locks the attachment member 4 to the fixation sleeve 3 while allowing relative axial movement. The fixation sleeve 3 includes two circumferential recesses 3e and 3f axially spaced apart a distance available for the movement of the fixation sleeve 3 relative to the attachment member 4 from the first position 5 to the second position 6. The circumferential recesses 3e and 3f on the inner surface of the fixation sleeve 3 may engage the protrusions 4h that protrude radially outwards from the proximal arms 4g of the holding section 4b. The circumferential recesses 3e and 3f are continuous recesses thereby allowing the insertion of the attachment member 3 at a plurality of rotational positions relative to the attachment member 4. The proximal circumferential recess 3f is axially positioned proximal to the distal circumferential recess 3e. The protrusions 4h on the attachment member may snap-fit into the distal circumferential recess 3e when the fixation sleeve 3 is in the first position 5 and the protrusions 4h may snap into the proximal circumferential recess 3f when the fixation sleeve 3 is in the second position 6. A circumferential rim 3g is axially positioned between the recesses 3e,3f and protrudes radially inward from the inner wall of the fixation sleeve 3. The protrusions 4h on the attachment member 4 need to pass the circumferential rim 3g when the fixation sleeve 3 moves from the first position to the second position or vice versa from the second position to the first position. The proximal arms 4g may flex during the movement across the rim 3g and the rim together with the flexible arms may provide a threshold force that needs to be overcome when axially moving the fixation sleeve 3 relative to the attachment member 4. A cross section of the assembly presented in Figure 11 in a plane D-D is presented in Figure 12. The key 3d on the inner surface of the fixation sleeve 3 engages the groove 4i between a pair of proximal arms 4g for axially guiding the fixation sleeve 3 with respect to the attachment member 4.

Fixation of the add-on module to the auto injector:

The assembly of the fixation sleeve 3 and the attachment member 4 in a longitudinal section in a plane taken different from Figure 11 is presented in Figures 13 and 14. The fixation sleeve 3 is in the first position in Figure 13 and the protrusion 4h on the distal arm 4g engages the distal circumferential recess 3e. The flexible arms 4e of the attachment member 4 are biased radially outwards such that the protrusions or hooks 4f at the distal end are all directed outwards providing an inner space 7 available for receiving the circumferential recess 10d of the auto injector. Each of the arms 4e engage a protrusion 3h that protrudes radially inward from the inner surface of the fixation sleeve 3. A sloped surface 3i is positioned distally from the protrusion 3h. The assembly with the fixation sleeve 3 in the second position is presented in Figure 14. The protrusion 4h has been moved over the rim 3g and engages the proximal circumferential recess 3f. The flexible arms 4e have been moved radially inward as the sloped surface 3i and protrusion 3h engage the flexible arms 4e.

Figure 15 presents the fixation sleeve 3 in the first position 5 and the auto injector with the circumferential recess 10d is inserted into the inner space between the arms 4e of the attachment member. The auto injector differs from the auto injector presented in Figures 1 and 2 in that the proximal end of the spring reel 20a engages the recess or passage 25f of the dispense indicator 25 for driving the dispense indicator. In the embodiment presented in Figures 1 and 2, the drive element 21 engages the dispense indicator 25. The distal end surfaces 4j (see Figure 11) may engage or abut the transparent cover 25b thereby guiding the attachment of the add-on module 2 and limiting the axial path. The flexible arms 4e may flex radially outwards as the protrusions 4f at the distal end engage the curved outer surface of the transparent cover 25b and may snap into the circumferential recess 10d of the auto injector. The recess 10d is a continuous recess such that the add-on module with the attachment member 4 may be positioned at any angular position around the longitudinal axis L of the auto injector as the arms 4e with the protrusions 4f may fit into the recess 10d at any angular position. The add-on module 2 presented in Figure 15 is not fixated to the distal end of the auto injector and may be removed as the fixation sleeve 3 is in the first position.

The flexible arms 4e are forced to move radially inwards by the protrusions 3h as the fixation sleeve 3 is moved to the second position 6 thereby providing an axial form fit between the protrusions 4f on the attachment member and the circumferential recess 10d on the auto injector, see Figure 16. The rotational locking between the add-on module 2 and the auto injector may be provided by a friction fit between each of the protrusions or hooks 4f on the arms 4e engaging the circumferential recess 10d. The fixation between the add-on module 2 and the auto injector is releasable by moving the fixation sleeve 3 from the second position (shown in Figure 16) back to the first position as shown in Figure 15. The arms 4e flex radially outwards and the form-fit and friction fit engagements between the attachment member 4 and the circumferential recess 10d is released such that the add-on module 2 may be removed and used on another auto injector after emptying the first auto injector.

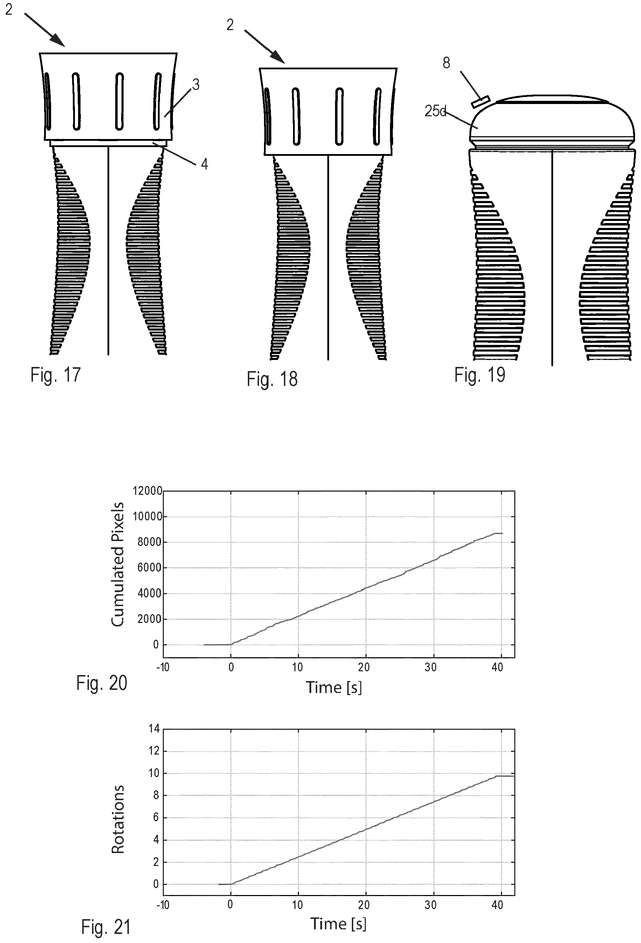

Another embodiment for the add-on module is presented in Figures 17 and 18. The fixation sleeve 3 in the first position does not fully cover the attachment member 4 whereas the fixation sleeve 3 fully covers the attachment member in the second position. The attachment member may be constructed from a different colored material, for example red, thereby providing an optical indication to the user that the fixation sleeve is in the first position where a red rim is visible for the user, and no red rim can be viewed after correct fixation of the add-on module.

**Optical flow sensor**

A schematic representation for the position of the optical flow sensor 8 with respect to the viewing window 25d is presented in Figure 19. The optical flow sensor includes a 2D sensor chip and is capable to view, detect or track the movement of the dispense indicator 25 located below the transparent cover 25b. The 2D sensor chip is oriented accordingly with the surface of the 2D sensor oriented towards the dispense indicator. The optical flow sensor 8 may be tilted with respect to the longitudinal axis of the auto injector. The optical flow sensor is part of, and integrated into the add-on module 2. The optical sensor may be attached to the attachment member 4, for example on a platform between two protrusions 4d (see Figure 10), or the optical flow sensor may be attached to a platform fixated to an inner surface of the fixation sleeve 3 (see Figure 10), or the optical flow sensor is integrated in a sensor holder attached to a printed circuit board that is supported by the attachment member 4, for example supported by the circumferential rim 4c.

The optical flow sensor may include a miniature low power optical navigation chip using laser based optical navigation technology enabling digital surface tracking of the dispense indicator through the viewing window. The optical flow sensor includes a laser light source, for example with a wavelength of 850 nm and a 2D image array providing a 2D sensor chip which measures changes in position by optically acquiring sequential surface images (frames) and mathematically determining the speed, the direction and the magnitude (distance) for the motion of the dispense indicator. The processor is programmed to run a mathematical algorithm enabling optical flow computation from optical flow sensor data such as the frames obtained from the sensor chip are transferred in distance, speed or number of rotations of the dispense indicator. An example of such an optical navigation chip may be the PAT9125EL optical flow sensor provided by Pixart Imaging Inc.

A relatively simple mathematical algorithm for computing the number of rotations (n) from subsequent frames that are shifted in the x direction by an amount (X) pixels and in the y direction by an amount (Y) pixels is presented in the following equation:

$$n = f * \sqrt{(X^2 + Y^2)}$$

The factor f is a calibration factor for transforming the respective pixel shifts X and Y in the number of rotations. The results are presented in Figure 20 for the pixel shifts and in Figure 21 for the calculation of the number of rotations.

The electronic circuit including the optical flow sensor may be activated or switched "on" when the fixation sleeve 3 is moved from the first to the second position and deactivated or switched "off" when the fixation sleeve 3 is in the first position or moved to the first position. An electronic switch integrated in the electronic circuit may be activated by the movement to the second position. Alternatively, two (or all) of the elastic arms 4e and the circumferential rim 4c may be made from an electrically conductive material such as a metal and the two conductive arms of the attachment member may be integrated in the electronic circuit. The circumferential recess 10 may include an electrically conductive surface and attaching the add-on module to the auto injector closes the electronic circuit as the conductive arms are connected via the conductive recess, whereas removing the add-on module from the auto injector opens the electronic circuit.

PART ANNOTATION

| | | | |
|---|---|---|---|
| 1 | Syringe holder | 11 | Reservoir, prefilled syringe |
| 2 | Add-on module | 11a | Needle shield |
| 3 | Fixation sleeve | 12 | Syringe holder |
| 3a | Distal end | 13 | Mechanic holder |

(continued)

| | | | |
|---|---|---|---|
| 3b | End wall | 13a | Holding/spring element |
| 3c | Side wall | 13b | Spring sleeve |
| 3d | Key | 14 | Needle cover sleeve |
| 3e | Distal circumferential recess | 14a | Arms |
| 3f | Proximal circumferential recess | 14b | Sleeve shaped section |
| 3g | Rim | 14c | Flange |
| 3h | Protrusion | 15 | Needle cover spring |
| 3i | Sloped surface | 16 | Cap remover |
| 4 | Attachment member, clamp | 17 | Switching sleeve |
| 4a | Gripping section | 18 | Locking sleeve |
| 4b | Holding section | 20a | Spring |
| 4c | Circumferential rim | 20b | Spring reel |
| 4d | Protrusion | 21 | Drive element |
| 4e | Flexible arm | 22 | Plunger rod |
| 4f | Hooks, protrusion | 23 | Coupling sleeve |
| 4g | Proximal arm | 23a | Holding elements |
| 4h | Protrusion | 24 | Bb |
| 4i | Groove | 25 | Dispense indicator |
| 4j | Distal end surface | 25b | Transparent cover |
| 5 | First position | 25c | Non transparent disk |
| 6 | Second position | 25d | Viewing window |
| 7 | Inner space | 25e | Elastic arm |
| 8 | Optical flow sensor | 25f | Recess/ passage |
| 10 | Housing | 25g | Skirt |
| 10a | Proximal housing | 25h | Bottom surface |
| 10b | Distal housing | 25i | Pattern |
| 10c | Viewing window | L | Longitudinal axis |
| 10d | Circumferential recess | | |
| 10e | Grip | | |

**Claims**

1. An add-on module (2) for an injection device comprising a dispense indicator (25) visible through a viewing window (10c) in a device housing (10) and the housing defining a longitudinal axis for the injection device, the add-on module (2) comprising:

   - an optical flow sensor (8) fixated to the add-on module and configured to track movement of the dispense indicator (25) relative to the housing (10) through the viewing window (10c),
   - a processor programmed to run an algorithm enabling optical flow computation from optical flow sensor data,

   wherein the add-on module (2) is adapted to be releasably fixated to the housing (10) and axially and rotationally fixated to the housing.

2. The add-on module according to claim 1 wherein the optical flow sensor (8) is adapted to track helical movement of the dispense indicator (25) relative to the housing around the longitudinal axis, or adapted to track an axial movement along the longitudinal axis.

3. The add-on module according to claim 1 wherein the optical flow sensor (8) is adapted to track a continuous rotational movement of the dispense indicator (25) around the longitudinal axis without axial movement relative to the housing (10).

4. The add-on module according to any of the previous claims wherein the add-on module (2) is adapted to be axially

12

fixated to a proximal end of the housing and rotationally fixated at any angular position around the longitudinal axis of the housing.

5. The add-on module according to any of the previous claims wherein the processor is adapted to computate the number of rotations of the dispense indicator (25).

6. The add-on module according to any of the previous claims wherein the optical flow sensor (8) is adapted to track movement of a dispense indicator (25) including non-alphanumeric patterns printed onto the surface or wherein the dispense indicator does not include a visible pattern on the surface.

7. The add-on module according to any of the previous claims wherein the viewing window (10c) is a transparent circumferential rim at the proximal end face of the injection device covering a disk shaped dispense indicator (25) rotatably mounted behind the transparent circumferential rim.

8. The add-on module according to any of the previous claims wherein the optical flow sensor (8) comprises a 2-Dimensional image sensor chip and an infrared laser light source and wherein the optical flow computation uses the displacement between subsequent images of the dispense indicator (25) taken by the image sensor chip.

9. The add-on module according to any of the previous claims further comprising:

- a fixation sleeve (3) adapted to be arranged over the proximal end of the housing (10),
- an attachment member (4) coupled to, and arranged within the fixation sleeve (3), the attachment member (4) being adapted to grip the proximal end of the housing,
wherein the fixation sleeve (3) is adapted to move relative to the attachment member (4) from a first position (5) allowing the dispense indicator (25) to be moved with respect to the attachment member (4) to a second position inhibiting relative movement,
wherein the attachment member (4) is adapted to be deformed during movement from the first to the second position for fixating the add-on module (2) to the proximal end of the housing (10).

10. The add-on module according to claim 9 wherein the attachment member (4) comprises an elastic arm (4e) adapted to flex towards the housing (10) of the injection device as the fixation sleeve (3) moves axially along the longitudinal axis from the first position (5) to the second position (6).

11. The add-on module according to claim 10 wherein a protrusion at the distal end of the elastic arm being adapted to engage a circumferential recess at the proximal end of the housing.

12. The add-on module according to claim 11 wherein the fixation sleeve (3) comprises a fixation member (3h) protruding from an inner surface of the fixation sleeve (3) comprising a ramped surface (3i) for engaging the elastic arm (4e).

13. A system comprising the add-on module (2) according to any of the previous claims and an injection device comprising the dispense indicator (25).

14. The system according to claim 13 wherein the injection device is an auto injector and wherein an injection spring (20a) advances a plunger rod (22) for expelling medicament from the injection device during an injection and wherein the injection spring (20a) is configured to continuously rotate the dispense indicator (25) during the injection.

15. The system according to claim 13 wherein the injection device is an injection pen with a dose dial sleeve that is manually rotated for setting of a dose and wherein the outer surface of the dose dial sleeve provides the dispense indicator (25).

Fig.1

25c
25b
25
20a
20b
13b
13
11
11a
23
23a
12
15
18
17
10c
13a
10b
21
14a
22
14
14b
14c
16
10a

Fig.2

11b  11  10b  10a  14a  17  18a  13  20b  25d

L

10d

14c  22  13a  23a  23  25  25b

Fig.3

25  25g  25e

25h

25e

25f  25i

Figure 4

Figure 5

Figure 6

Figure 7

Figure 8

Fig. 9

Fig. 10

Fig. 11

Fig. 12

D-D

Fig. 13

Fig. 14

Fig. 15

Fig. 16

Fig. 17

Fig. 18

Fig. 19

Fig. 20

Fig. 21

**Europäisches Patentamt**
**European Patent Office**
**Office européen des brevets**

# EUROPEAN SEARCH REPORT

Application Number

EP 23 19 5311

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/052819 A1 (SCHABBACH MICHAEL [DE]) 25 February 2021 (2021-02-25) | 1-7,9-15 | INV. A61M5/20 |
| Y | * figures 1, 5, 10, 11A-B, 13, 14, 15, 16, 17, 18 * <br> * paragraphs [0056] – [0158] * | 8-12 | A61M5/24 |
| X | EP 3 369 446 A2 (SANOFI AVENTIS DEUTSCHLAND [DE]) 5 September 2018 (2018-09-05) | 1-8, 13-15 | |
| Y | * figures 1a-b, 2, 3, 4, 5, 6, 7, 8 * <br> * paragraphs [0019] – [0058] * | 8-12 | |
| X | US 2020/327974 A1 (MELZI ILARIO [IT]) 15 October 2020 (2020-10-15) | 1-7,9-15 | |
| Y | * figures 1A-B, 2a-b, 3, 4, 5A-C, 6A-C, 7A-C, 11, 12 * <br> * paragraphs [0048] – [0134] * | 8-12 | |
| A | US 2023/021831 A1 (KALBERMATTER GABRIEL [CH] ET AL) 26 January 2023 (2023-01-26) * the whole document * | 1-15 | |
| A | US 2019/201627 A1 (HELMER MICHAEL [DE] ET AL) 4 July 2019 (2019-07-04) * the whole document * | 1-15 | **TECHNICAL FIELDS SEARCHED (IPC)** <br> A61M |
| A | US 2014/194825 A1 (NIELSEN OLE CHRISTIAN [DK] ET AL) 10 July 2014 (2014-07-10) * the whole document * | 1-15 | |

The present search report has been drawn up for all claims

| Place of search | Date of completion of the search | Examiner |
|---|---|---|
| Munich | 21 December 2023 | Benes, Václav |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# EP 4 520 365 A1

## ANNEX TO THE EUROPEAN SEARCH REPORT
## ON EUROPEAN PATENT APPLICATION NO.

EP 23 19 5311

This annex lists the patent family members relating to the patent documents cited in the above-mentioned European search report.
The members are as contained in the European Patent Office EDP file on
The European Patent Office is in no way liable for these particulars which are merely given for the purpose of information.

21-12-2023

| Patent document cited in search report | | | Publication date | Patent family member(s) | | | Publication date |
|---|---|---|---|---|---|---|---|
| US 2021052819 | A1 | | 25-02-2021 | CN | 111511422 | A | 07-08-2020 |
| | | | | CN | 116942965 | A | 27-10-2023 |
| | | | | EP | 3731900 | A1 | 04-11-2020 |
| | | | | EP | 4241810 | A2 | 13-09-2023 |
| | | | | JP | 2021508553 | A | 11-03-2021 |
| | | | | US | 2021052819 | A1 | 25-02-2021 |
| | | | | WO | 2019129621 | A1 | 04-07-2019 |
| EP 3369446 | A2 | | 05-09-2018 | CN | 104902944 | A | 09-09-2015 |
| | | | | CN | 109381776 | A | 26-02-2019 |
| | | | | DK | 2945665 | T3 | 14-06-2018 |
| | | | | EP | 2945665 | A1 | 25-11-2015 |
| | | | | EP | 3369446 | A2 | 05-09-2018 |
| | | | | HK | 1211511 | A1 | 27-05-2016 |
| | | | | HK | 1256894 | A1 | 04-10-2019 |
| | | | | JP | 6316312 | B2 | 25-04-2018 |
| | | | | JP | 2016502899 | A | 01-02-2016 |
| | | | | TR | 201807770 | T4 | 21-06-2018 |
| | | | | US | 2016015902 | A1 | 21-01-2016 |
| | | | | US | 2017340826 | A1 | 30-11-2017 |
| | | | | WO | 2014111340 | A1 | 24-07-2014 |
| US 2020327974 | A1 | | 15-10-2020 | CN | 111712284 | A | 25-09-2020 |
| | | | | CN | 116196508 | A | 02-06-2023 |
| | | | | EP | 3727519 | A1 | 28-10-2020 |
| | | | | EP | 4241809 | A2 | 13-09-2023 |
| | | | | JP | 2021506510 | A | 22-02-2021 |
| | | | | JP | 2023130488 | A | 20-09-2023 |
| | | | | US | 2020327974 | A1 | 15-10-2020 |
| | | | | US | 2023335251 | A1 | 19-10-2023 |
| | | | | WO | 2019121607 | A1 | 27-06-2019 |
| US 2023021831 | A1 | | 26-01-2023 | CH | 715791 | A2 | 31-07-2020 |
| | | | | CN | 115279435 | A | 01-11-2022 |
| | | | | EP | 3930793 | A1 | 05-01-2022 |
| | | | | EP | 4289456 | A2 | 13-12-2023 |
| | | | | US | 2023021831 | A1 | 26-01-2023 |
| | | | | WO | 2021191095 | A1 | 30-09-2021 |
| US 2019201627 | A1 | | 04-07-2019 | CN | 109641103 | A | 16-04-2019 |
| | | | | EP | 3506966 | A1 | 10-07-2019 |
| | | | | JP | 7066713 | B2 | 13-05-2022 |
| | | | | JP | 2019529009 | A | 17-10-2019 |
| | | | | US | 2019201627 | A1 | 04-07-2019 |
| | | | | WO | 2018041798 | A1 | 08-03-2018 |

EPO FORM P0459

For more details about this annex : see Official Journal of the European Patent Office, No. 12/82

21

## ANNEX TO THE EUROPEAN SEARCH REPORT
## ON EUROPEAN PATENT APPLICATION NO.

EP 23 19 5311

This annex lists the patent family members relating to the patent documents cited in the above-mentioned European search report.
The members are as contained in the European Patent Office EDP file on
The European Patent Office is in no way liable for these particulars which are merely given for the purpose of information.

21-12-2023

| Patent document cited in search report | Publication date | Patent family member(s) | | Publication date |
|---|---|---|---|---|
| US 2014194825 A1 | 10-07-2014 | CN | 103702699 A | 02-04-2014 |
| | | EP | 2729202 A1 | 14-05-2014 |
| | | JP | 6106666 B2 | 05-04-2017 |
| | | JP | 2014520584 A | 25-08-2014 |
| | | US | 2014194825 A1 | 10-07-2014 |
| | | WO | 2013004844 A1 | 10-01-2013 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10043093 B2
- WO 2013120775 A
- US 20220016352 A1